# EUROPEAN PATENT SPECIFICATION

(11) **EP 0 356 137 B1**
(45) Date of publication and mention of the grant of the patent: **26.10.1994**
(21) Application number: 89308341.0
(22) Date of filing: 17.08.1989
(51) Int. Cl.: C07C 323/41, A61K 31/645

(54) **Carbonates of 3-demethylthiocolchicine and N-acyl analogs**
3-Demethylthiocolchicincarbonate und N-Acylanaloge
Carbonates de la 3-deméthylthiocolchicine et des analogues N-acylés

(30) Priority: 24.08.1988 US 235907
(43) Date of publication of application: 28.02.1990
(73) Proprietor: Advance Biofactures Corporation, Lynbrook New York 11563 (US)
(72) Inventor: Brossi, Arnold, Dr., Bethesda Maryland 20817 (US)
(74) Representative: Bankes, Stephen Charles Digby

(56) References cited:
- FR-A- 1 099 138
- US-A- 2 820 029
- HELVETICA CHIMICA ACTA, vol. 68, no. 3, May 15, 1985, pages 371-580, Basel, CH; P. KEREKES et al, "Esters of 1-O-demethylthiocolchicines: formation of isomers in chloroform solution"
- CHEMICAL ABSTRACTS, vol. 112, no. 1, 1 February 1990, pages 525-526, abstract no. 21187n, Columbus, Ohio, US; A. BROSSI, "3-Demethylthiocolchicine derivatives for treatment of gout, Mediterranean fever, and liver disorders"; & US-A-235 907
- HETEROCYCLES, vol. 28, no. 1, 1989, pages 365-372, Tokyo, JP; A. MUZAFFAR et al, "Phenolic congeners of colchicine: preparation and characterization of phenolic and catecholic analogues of colchicine, colchiceine and thiocolchicine"

## Description

The present invention relates to esters of 3-demethylthiocolchicine and N-acyl analogs as substitutes of colchicine in diseases treated with colchicine, particularly liver disorders. Moreover, the invention is concerned with pharmaceutical compositions of the esters of 3-demethylthiocolchicine and N-acyl derivatives, methods of treating diseases such as gout, Mediterranean fever and liver disorders and a process for the preparation of the ester compounds.

3-demethylthiocolchicine has broad-spectrum antitumor properties and has been reported to inhibit migration of amyloids (A. Brossi et al, Med. Res. Reviews, 1988, Vol. 8, pp. 77-94). Therefore, 3-demethylthiocolchicine is a substitute of colchicine having an improved therapeutic index. Although 3-demethylthiocolchicine is a known compound, the ester derivatives thereof having similar biological properties have not been reported.

U.S. Patent 4,692,463 by Brossi, which is not prior art against the instant invention, describes the finding of potent antiinflammatory properties in 2,3-didemethylcolchicine and its derivatives. Although 3-demethylthiocolchicine is disclosed, the esters thereof are not reported.

U.S. Patent 3,997,506 by Dugat discloses anti-mitotic and anti-gout derivatives of thiocolchicine.

U.S. Patent 3,816,396 discloses a process for the preparation of glucoside derivatives of thiocolchicine.

U.S. Patent 2,820,029 by Muller et al discloses thio-derivatives of colchiceine compounds and a process of making the same.

A publication by Kerekes et al entitled "Esters of 1-0-Demethylthiocolchicines: Formation of Isomers in Chloroform Solution", Helvetica Chimica Acta, Vol. 68, 1985, p. 579, discloses 3-0-Acetyl-10-demethoxy-3-0-demethyl-10(methylthio) colchicine.

A French language publication by Velluz et al entitled "La thiocolchicine. III. - Etude de quelques S-alcoyl-thiocolchiceines", Memoires presentes a la Societe Chimique, pp. 194-197, discloses the preparation and study of thiocolchicine.

This invention relates to novel esters of 3-demethylthiocolchicine and N-acyl analogs thereof. The invention further relates to pharmaceutical compositions of these 3-demethylthiocolchine and N-acyl analogs as well as their utility in the treatment of diseases treated with colchicine, particularly liver disorders. Moreover, the invention is also directed to a process for preparing novel esters of 3-demethylthiocolchine and N-acyl analogs.

The compounds of the invention will hereinafter be described in greater detail.

### Description of Esters of 3-demethylthiocolchicine and N-acyl analogs thereof.

More particularly, the present invention relates to esters of 3-demethylthiocolchicine and N-acyl analogs thereof having the formula: 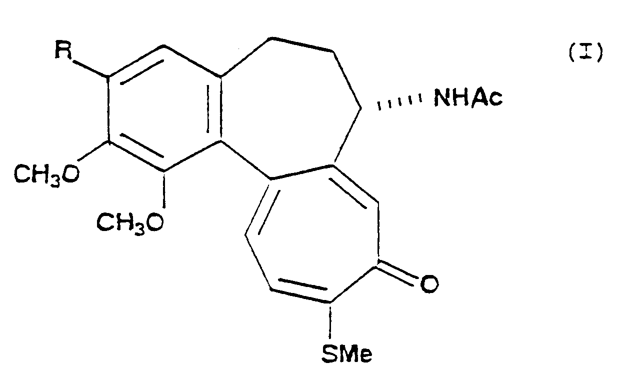
wherein R is an O-COOalkyl group, the alkyl group having 1 to 4 carbon atoms, and the Ac group is acetyl or its higher homologs, haloacetyl, or alkoxyacetyl.

Representative examples of alkyl groups having 1 to 4 carbon atoms include methyl, ethyl, n-propyl, isopropyl, n-butyl, isobutyl, tert-butyl and sec-butyl.

Higher homologs of the acetyl group include those acetyl groups containing 1 to 8 carbon atoms including saturated fatty acids, and more particularly those acetyl groups containing 1 to 4 carbon atoms.

Representative examples of the alkoxyacetyl group include those alkoxy groups containing 1 to 8 carbon atoms, particularly 1 to 4 carbon atoms.

Suitable examples of the haloacetyl group include mono-substituted and tri-substituted haloacetyl groups including fluoroacetyl, chloracetyl, bromoacetyl, and trifluoroacetyl wherein the acetyl group is as defined above. Preferably, the mono and trifluoroacetyl groups are used.

The preferred compounds of the invention are 3-ethoxycarbonyl-3-demethylthiocolchicine and 3-butoxycarbonyl-3-demethylthiocolchicine.

3-demethylcolchicine is a natural product (H.G. Capraro and A. Brossi, The Alkaloids, Vol. 23, Academic Press, 1984, p.10) which occurs, in addition to colchicine, in a variety of plant species such as Colchicum autumnale and Gloriosa superba. 3-demethylcolchicine may be converted by reaction with sodium methylthiolate into 3-demethylthiocolchicine which on further reaction at the OH position on the phenol reacts with acylating agents such as butyric anhydride or esterification agents such as ethyl chloroformate to produce the ester derivatives shown in formula (I), wherein R is defined above.

3-demethylthiocolchicine prepared from the commercial drug thiocolchicoside (e.g. obtained from Roussel-Uclaf) by treatment with 80% phosphoric acid (P.N. Sharma and A. Brossi, Heterocycles, 1983, Vol. 20, p.1587) has potent antitumor activity in experimental animals (P. Kerekes, P.N. Sharma, A. Brossi, C.F. Chignell and F.R. Quinn, J. Med. Chem., 1985, Vol. 28, p.1204) and inhibits amyloid migration (M. Ravid, M. Gotfried and A. Brossi, "Amyloidosis" 1988, Plenum Press, New York, in press). These activities in this series of compounds parallel their affinity for tubulin and, therefore, a tublin-affinity test allows an assessment of the potential medical uses of these compounds (A. Brossi, H.J.C. Yeh, M. Chrzanowska, J. Wolff, E. Hamel, C.M. Lin, F. Quinn, M. Suffness and J.V. Silverton, Med. Res. Reviews, 1988, Vol. 8, pps.77-94).

Using such data, which are shown in Example 9, it is clear that the ester derivatives covered by formula (I) have similar biological effects as the parent phenol and are at least as potent as colchicine. 3-demethylthiocolchicine has emerged from elaborate study as a less toxic analog of colchicine with a larger therapeutic index (M. Ravid, M. Gotfried and A. Brossi, "Amyloidosis", 1988, Plenum Press, New York, in press) and its ester derivatives can, therefore, be considered to represent useful compounds for the treatment of gout, Mediterranean fever (FMF) and liver disorders such as cirrhosis of the liver, tumors and inflammation of unspecific origin.

The ester derivatives covered by formula (I) above are prepared from natural (-)-3-demethylcolchicine (H.G. Capraro and A Brossi, "The Alkaloids", Vol. 23, Academic Press, 1984, p.10.) via (-)-3-demethylthiocolchicine obtained from the former by reaction with sodium methylthiolate in water. 3-demethylcolchicine also can be obtained from colchicoside as a natural alkaloid (H.G. Capraro and A. Brossi, "The Alkaloids", Vol. 23, Academic Press, 1984, p.10) which can be converted into 3-demethylcolchicine by treatment with 80% phosphoric acid (M. Rosner, H.G. Capraro, A.E. Jacobson, L. Atwell, A. Brossi, M.A. Torio, T.H. Williams, R.H. Sik and C.F. Chignell, J. Med. Chem. 1981, Vol. 24, p.257.)

Methods for preparing esters of 3-demethylthiocolchicine and N-acyl derivatives thereof will hereinafter be described in greater detail.

### Methods for preparing esters of 3-demethylthiocolchicine and N-acyl derivatives thereof.

Accordingly, the invention is further directed to a process for the preparation of 3-demethylthiocolchicine or the esters thereof from 3-demethylcolchicine obtained from colchicoside. 3-demethylthiocolchicine can be obtained from 3-demethylcolchicine by treatment with an alkaline salt of methyl mercaptane commercially available as sodium or potassium methylthiolate and the reaction occurs in water at temperatures between about 0 and 30°C, preferably at room temperature. Material prepared by this route is converted into the ester analogs as shown in the following reaction scheme. 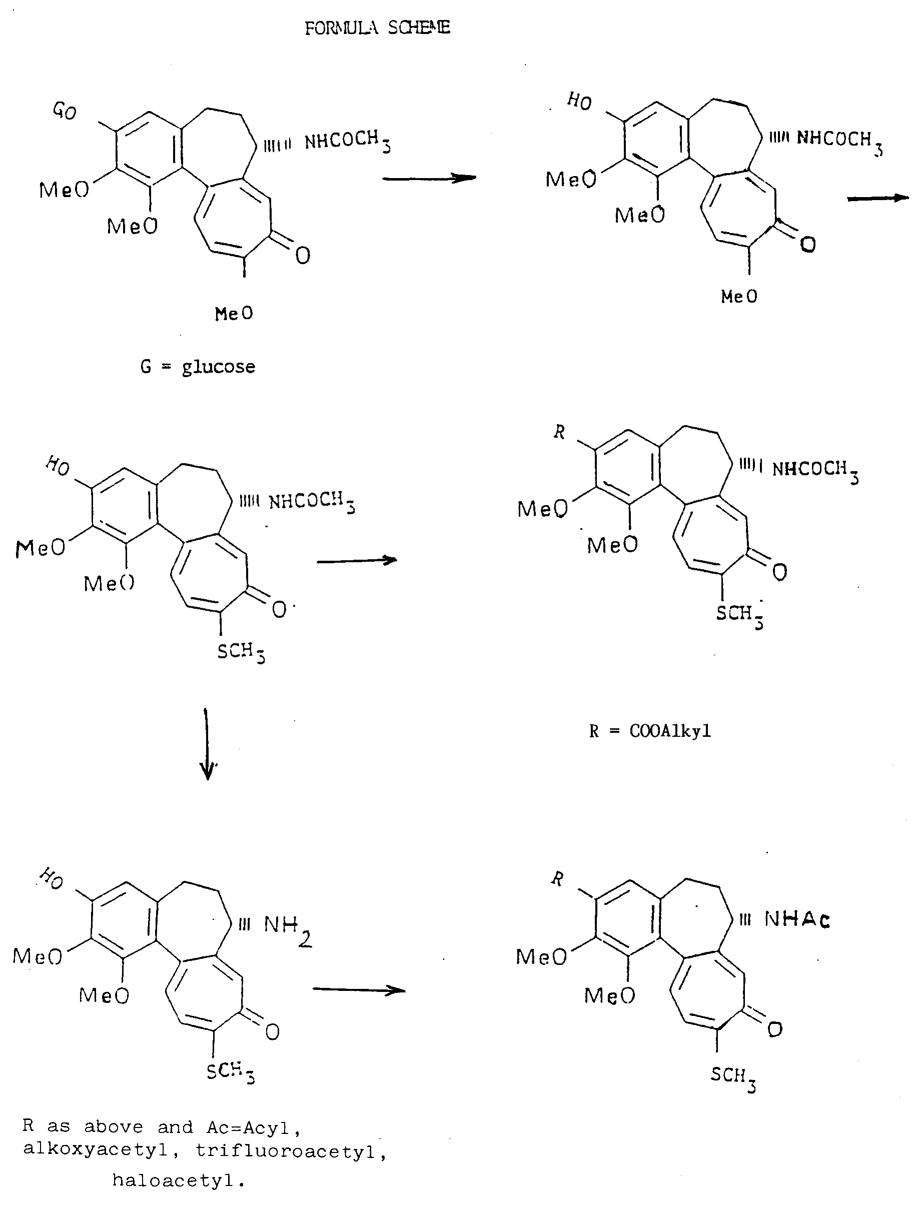
The esterification may proceed via conventional techniques, such as treating the phenol with acid chloride in the presence of sodium hydroxide (Schotten-Baumann reaction), or with acylanhydride in the presence of pyridine or triethylamine, or with alkylchloroformate in the presence of pyridine.

The ester analogs are neutral materials, which can be crystallized from solvents such as acetone, ethylacetate, isopropyl ether, chloroform-pentane, etc. They also can be chromatographed on aluminum oxide or silica gel and eluted with mixtures of solvents such as methylenechloride/methanol 95:5 or 90:10 etc. The compounds are optically active and have the same configuration as colchicine.

The acetyl group in 3-demethylthiocolchicine can be replaced by other acyl groups such as propionyl, butyryl, trifluoroacetyl, through acid hydrolysis of 3-demethylthiocolchicine with 20% sulfuric or hydrochloric acid (e.g. for about 3 hours). These esteramides on hydrolysis with potassium carbonate in aqueous methanol afford the corresponding acyldeacetylthiocolchicine analogs which can be esterified as mentioned above. For example, N-butryryldeacetyl-3-demethylthiolcolchicine prepared by this sequence of reactions affords after treatment with acetic anhydride in pyridine the corresponding acetate. Procedures involving acid hydrolysis of colchicine with sulfuric acid and treatment with anhydrides are set forth in Dumont et al, Journal of Organic Chemistry, June 27, 1986, pp 2515-2521, by the American Chemical Society, which is herein incorporated by reference.

The following Examples are intended to illustrate processes for preparing the claimed invention and will enable others skilled in the art to understand the invention more completely. However, the invention should not be interpreted as being limited to only these representative examples.

### EXAMPLE 1

A mixture of 250 mg of colchicoside and 2 ml of 85-86% H₃PO₄ is stirred at 25°C for 24 hours. The yellow solution is diluted with 10 ml of water, adjusted to pH 5 by addition of 2N NaOH and extracted with methylene chloride. The extracts are dried (Na₂SO₄), evaporated and chromatographed on Al₂O₃ (basic, Alfa Division, CH₂C₁₂/MeOH = 95:5). The appropriate fractions after evaporation of solvent and crystallization from acetone afford 180 mg (89%) of 3-demethylcolchicine: mp 176-177°C (resolidification at 205-210°C and 2nd mp 270°C (dec.), [α]_{D} = 151° (solvate crystal, CHCl₃).

3-demethylcolchicine (600 mg) in water (6 ml) was added with sodium methanethiolate (Fluka, 806 mg). The reaction was stirred for 24 hours, diluted with 2% acetic acid (15 ml) and then extracted with chloroform (3 x 100 ml) to give a yellow powder (530 mg). Crystallization from acetone gave 3-demethylthiocolchicine. mp 310°C, [α]_{D} = -259° (c = 0.2, CHCl₃).

### EXAMPLE 2

3-demethylthiocolchicine (100 mg) is dissolved in methylene chloride (3 ml) and triethylamine (0.5 ml) is added with ethylchloroformate (0.5 ml) and the reaction mixture is left under stirring at room temperature for 3 hours. The reaction mixture is then diluted with methylene chloride (20 ml), washed with 2N HCl, saturated sodium carbonate solution and brine, to afford after evaporation of solvent and crystallization from ethylacetate the ethylcarbonate of 3-dimethylthiocolohicine. mp 249°C, [α]_{D} = -128° (c = 0.1, CHCl₃).

### EXAMPLE 3

3-demethylthiocolchicine (100 mg) in pyridine (3 ml) is added with butylchloroformate (0.2 ml) under ice cooling. After 1 hour standing, the reaction mixture is adjusted to pH 5 by addition of 2N HCl and extracted with chloroform. After washing with 5% HCl, water and sodium carbonate solution and drying over MgSO₄, 108 mg of 3-butoxycarbonyl-3demethylthiocolchicine is obtained as a yellow powder. mp 212-215°C; [α]_{D} = -110.8° (c = 0.3, CHCl₃).

The compositions of the invention will hereinafter be described in greater detail.

### Compositions containing esters of 3-demethylthiocolchicine and N-acyl derivatives thereof.

The compounds of the present invention can be made into pharmaceutical compositions by combination with appropriate medical carriers or diluents. For example, the compounds can be mixed with starch, sugars and other inert ingredients commonly used to make tablets and used as tablets for oral application. The compounds can also be dissolved in oils, propyleneglycol or other solvents commonly used to prepare injectable solutions. Such preparations can be used for the treatment of gout, FMF, liver disorders, such as liver cirrhosis and as antitumor and antiinflammatory agents. For topical application, they can be formulated as ointments or creams.

More specifically, the compounds of the present invention may be formulated into preparations in solid, semisolid, liquid or gaseous form such as tablets, capsules, powders, granules, ointments, solutions, suppositories, and injections, in usual ways for oral or parenteral administration.

The following methods and excipients are merely exemplary and in no way limit the invention.

The compounds of the present invention in pharmaceutical dosage forms may be used in the form of their pharmaceutically acceptable salts, and also may be used alone or in appropriate association, as well as in combination with other pharmaceutically active compounds.

The compounds in the case of oral preparation may be used alone or combined with appropriate additives to make tablets, powders, granules or capsules, e.g. with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethylcellulose; with lubricants such as talc or magnesium stearate; and if desired, with diluents, buffering agents, moistening agents, preservatives and flavoring agents.

The compounds of the present invention may be formulated into preparations for injections by dissolving, suspending or emulsifying them in aqueous solvents such as normal saline, Dextrose 5%, or non-aqueous solvent, such as vegetable oil, synthetic aliphatic acid glycerides, esters of higher aliphatic acids or propylene glycol; and if desired, with conventional additives such as solubilizers, isotonic agents, suspending agents, emulsifying agents, stabilizers and preservatives.

The compounds of the invention may be combined with other compounds such as with other antitumor agents.

The following example further illustrates the composition of the present invention and will enable others skilled in the art to understand the invention more completely. It is understood that the invention is not limited to the Example below.

### EXAMPLE 4

2 mg of the active ingredient is combined with 187 grams of microcrystalline cellulose as a carrier, 9 mg of stearic acid and 2 mg of colloidal silica. These materials are pressed to form a tablet.

Methods of treating various diseases or disorders with the compound or composition of the invention will hereinafter be described in greater detail.

### Methods for treating diseases or disorders.

The desirable dose of the compounds of the present invention varies with the subject, drug form, method and period of administration. However, in order to obtain desirable effects, generally it is recommended to administer 0.1mg to 40 mg/kg, preferably 0.5 to 10 mg/kg, body weight of the compounds of the present invention. In terms of composition, compounds should be present between 0.1 to 100% by weight.

### Biological Data

The following examples further illustrate that the ester compounds of present invention have potential as antimicrotubule agents in mammals such as humans and will enable others skilled in the art to understand the invention more completely. Of course, it is understood that the invention is not limited to the Examples below.

### EXAMPLE 5

Compounds were evaluated both as inhibitors of tubulin polymerization and as inhibitors of the binding of radiolabeled colchicine to tubulin. The claimed compounds were compared to colchicine, 3-demethylcolchicine, thiocolchicine, and 3-demethylcolchicine. Additional procedures for evaluating these compounds are set forth by H.G. Capraro and A. Brossi, "The Alkaloids", Vol. 23, Academic Press, 1984, pp. 48-57, which is herein incorporated by reference. Moreover, this publication further shows that a good correlation exists between compounds active in vivo and their tubulin affinity in vitro.

| Compound | Inhibition of tubulin polymerization IC₅₀( M) | Inhibition of [³H]colchicine binding % inhibition |
|---|---|---|
| colchicine (Ref) | 2-3 | 27 |
| 3-demethylcolchicine (Ref) | 2-3 | 23 |
| thiocolchicine (Ref) | 1-2 | 54 |
| 3-demethylthiocolchicine (Ref) | 1-2 | 36 |
| 3-acetyl-3-demethyl-thiocolchicine (Ref) | 2-3 | 25 |
| 3-ethoxycarbonyl-3-demethylthiocolchicine | 1-2 | 49 |

The polymerization assay includes a drug-tubulin preincubation prior to GTP addition to allow slow binding drugs like colchicine to interact with the protein. The value in the table indicates the range of drug concentration required to obtain 50% inhibition of the extent of the polymerization reaction. Any value below 10 µM indicates a potent antimicrotubule agent with significant antimitotic potential.

The colchicine binding assay was performed without a drug-tubulin preincubation. The potential inhibitors (including nonradiolabeled colchicine) were present at a 1:1 molar ratio with the radiolabeled colchicine. (A concentration of radiolabeled colchicine which is rate-limiting is used. As a consequence, equimolar nonradiolabeled colchicine inhibits less than 50%.) The values in the table thus reflect both the affinity of the agents for tubulin relative to that of colchicine and relative binding rates of the agents relative to that of colchicine. Any inhibitory activity at all at the low drug concentrations used in this experiment (5 µM) indicates significant binding at the colchicine site.

The invention is also directed to a method of treating gout, Mediterranean fever and liver disorders which comprises administering to a patient a pharmaceutically effective amount of the instant composition. Administration is preferably by injection, orally, or with cream or ointment.

### EXAMPLE 6

A tablet is first formulated and prepared as in Example 4. The tablet is orally administered to a patient and represents a typical daily dosage.

## Claims (Claims for the following Contracting State(s): AT, BE, CH, DE, FR, GB, IT, LI, LU, NL, SE)

1. An ester compound of 3-demethylthiocolchicine or an N-acyl analogs thereof having the formula: 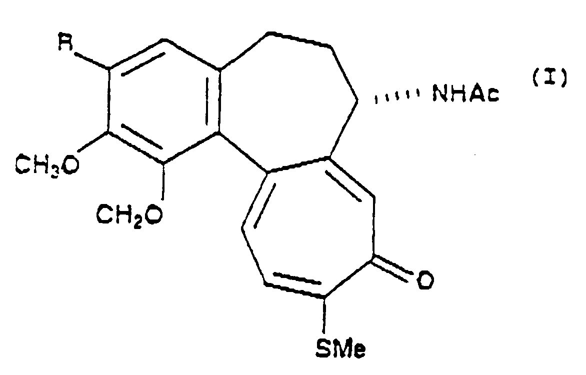 wherein R is an O-COOalkyl group, the alkyl group having 1 to 4 carbon atoms, and the Ac group is acetyl or its higher homologs, haloacetyl or alkoxyacetyl.

2. A compound according to claim 1, which is 3-ethoxycarbonyl-3-demethylthiocolchicine.

3. A compound of claim 1, which is 3-butoxycarbonyl-3-demethylthiocolchicine.

4. A compound according to any preceding claim for use in the treatment of gout.

5. A compound according to any one of claims 1 to 3, for use in the treatment of mediterranean fever.

6. A compound according to any one of claims 1 to 3, for use in the treatment of liver disorders.

7. A compound according to claim 6, for use in the treatment of cirrhosis, tumor growth or inflammation of the liver.

8. A pharmaceutical composition, which comprises a pharmaceutically effective amount of a compound according to any preceding claim and a pharmaceutically acceptable carrier therefor.

9. A pharmaceutical composition according to claim 8, in injectable form.

10. A pharmaceutical composition according to claim 8, in orally administrable form.

11. A pharmaceutical composition according to claim 8, in the form of a cream or ointment.

12. A composition according to any one of claims 8 to 11, in dosage units of 0.5 to 10 mg/kg body weight of the patient.

13. A process for the preparation of an ester compound of 3-demethylthiocolchicine, which comprises:
(a) reacting colchicoside (i) with phosphoric acid to form 3-demethylcolchicine (ii) 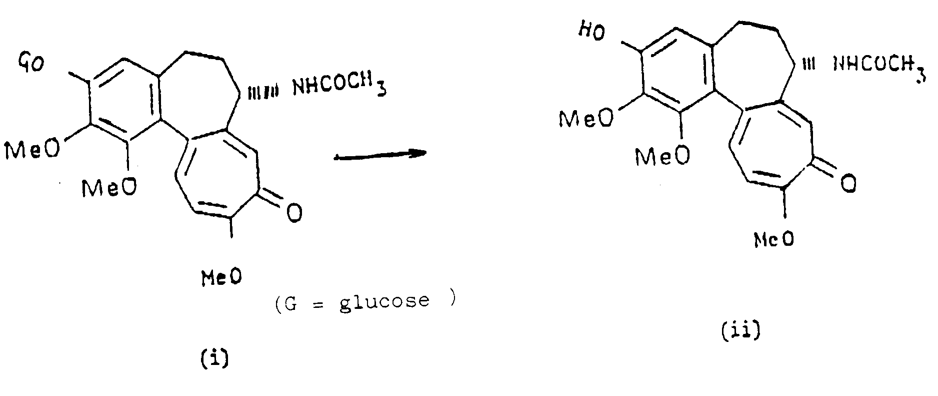
(b) reacting 3-demethylcolchicine (ii) with an alkaline salt of methyl mercaptan and water at room temperature to form 3-demethylthiocolchicine (iii), and 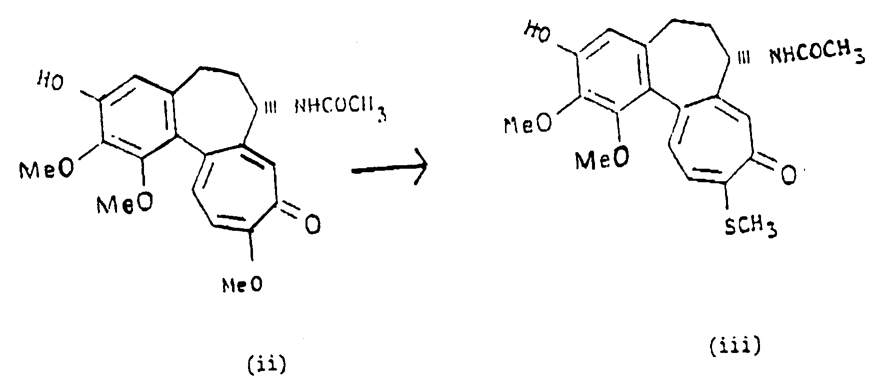
(c) esterifying 3-demethylthiocolchicine (iii) to form an ester analog thereof (iv) 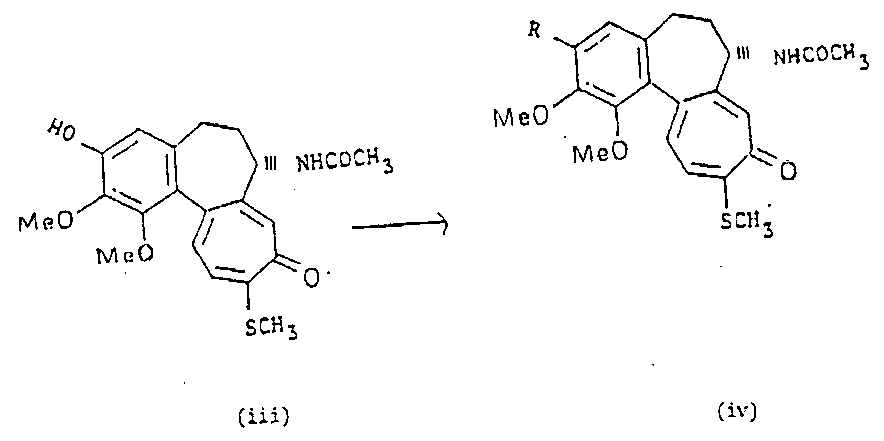 wherein R is an O-COOalkyl group, the alkyl group having 1 to 4 carbon atoms.

14. A process for the preparation of a compound according to claim 1, which comprises:
a) reacting colchicoside (i) with phosphoric acid to form 3-demethylcolchicine (ii) 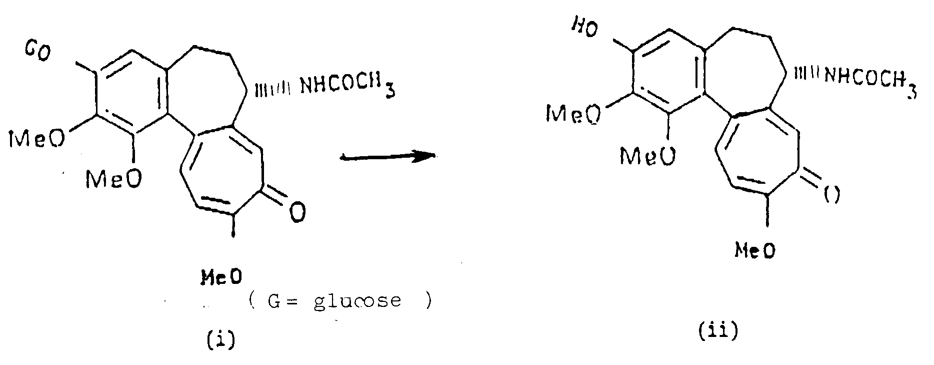
b) reacting 3-demethylcolchicine (ii) with an alkaline salt of methyl mercaptone and water at room temperature to form 3-demethylthiocolchicine (iii) 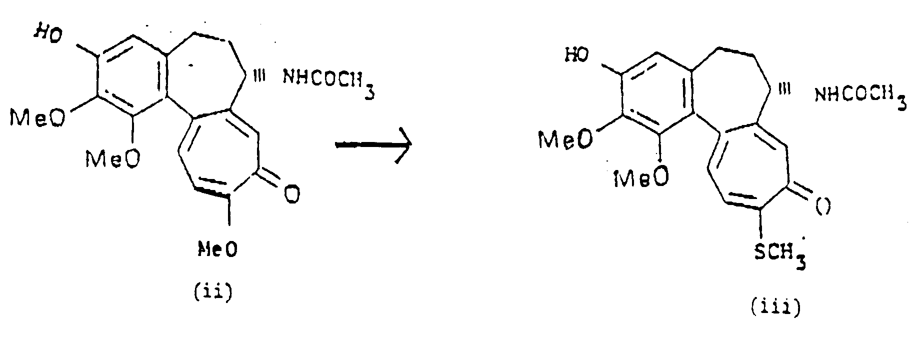
c) hydrolyzing 3-demethylthiocolchicine (iii) to remove the acetyl group in order to form compound (v), and 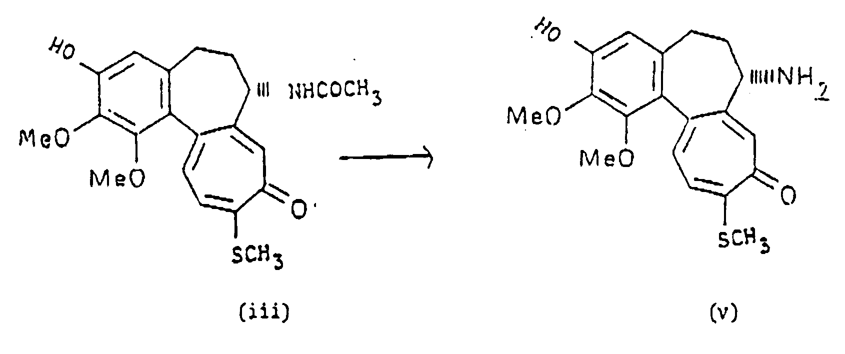
d) N-acylating and esterifying compound (v) to form an ester analog of 3-demethylthiocolchicine (vi) 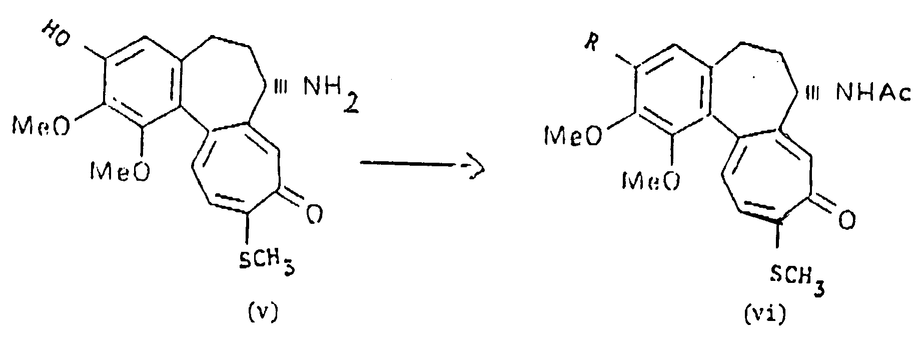 wherein R is an O-COOalkyl group, the alkyl group having 1 to 4 carbon atoms, and the Ac group is acetyl or its higher homologs, haloacetyl, or alkoxyacetyl.

## Claims (Claims for the following Contracting State(s): ES, GR)

1. A process for the preparation of an ester of 3-demethylthiocolchicine, which comprises:
a) reacting colchicoside (i) with phosphoric acid to form 3-demethylcolchicine (ii) 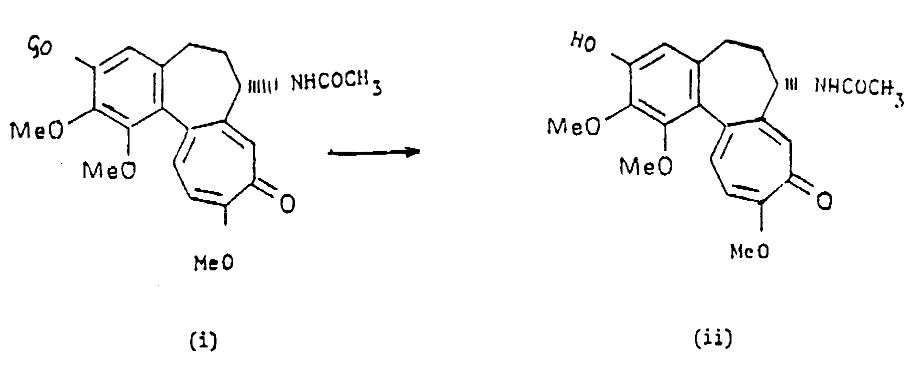
b) reacting 3-demethylcolchicine (ii) with an alkaline salt of methyl mercaptan and water at room temperature to form 3-demethylthiocolchicine (iii), and 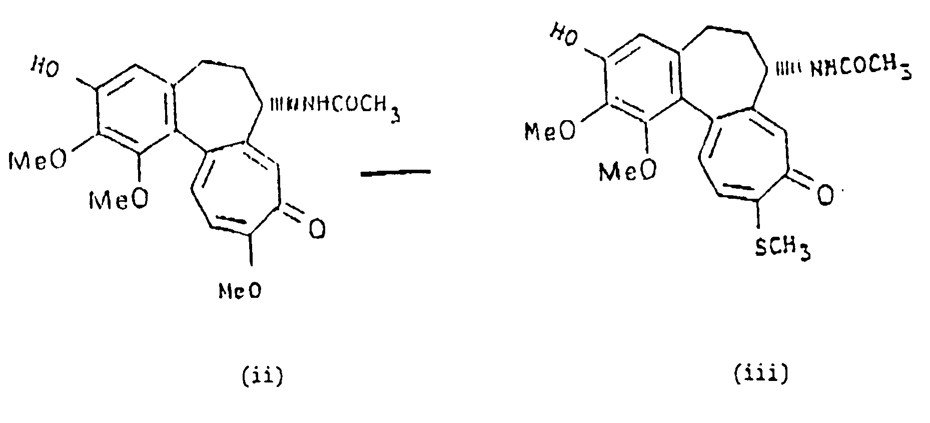
c) esterifying 3-demethylthiocolchicine (iii) to form an ester analog thereof (iv) 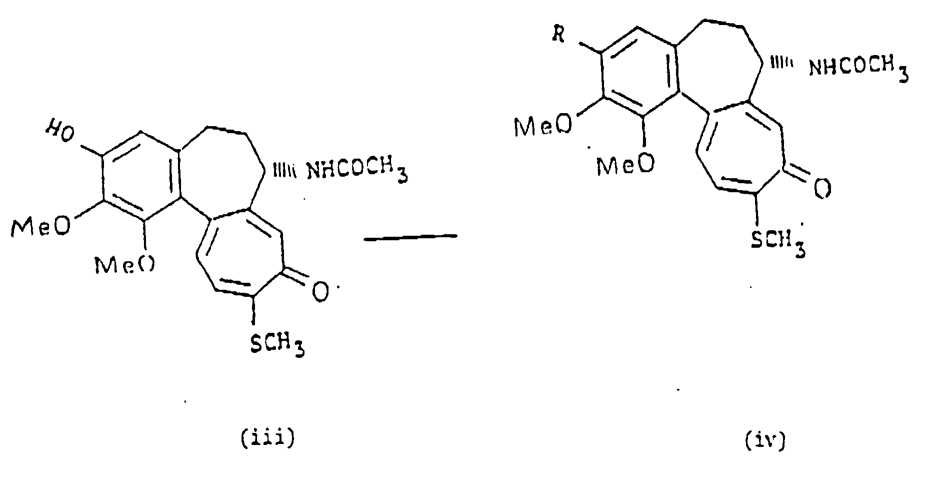 wherein R is an O-COOalkyl group, the alkyl group having 1 to 4 carbon atoms.

2. A process for the preparation of an ester of 3-demethylthiocolchicine, which comprises:
a) reacting colchicoside (i) with phosphoric acid to form 3-demethylcolchicane (ii) 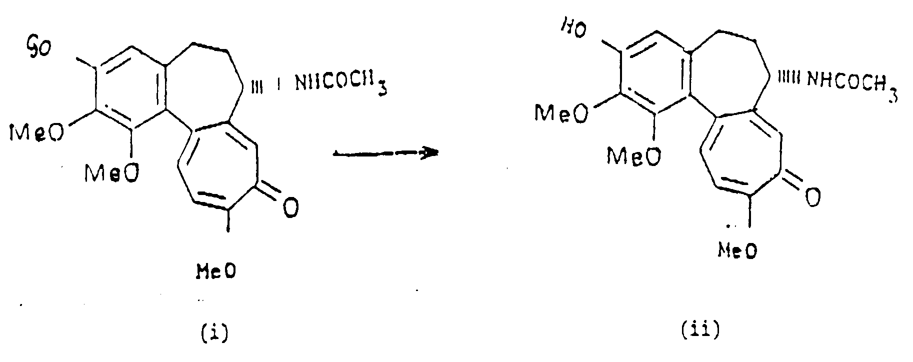
b) reacting 3-demethylcolchicine (ii) with an alkaline salt of methyl mercaptone and water at room temperature to form 3-demethylthiolchicine (iii) 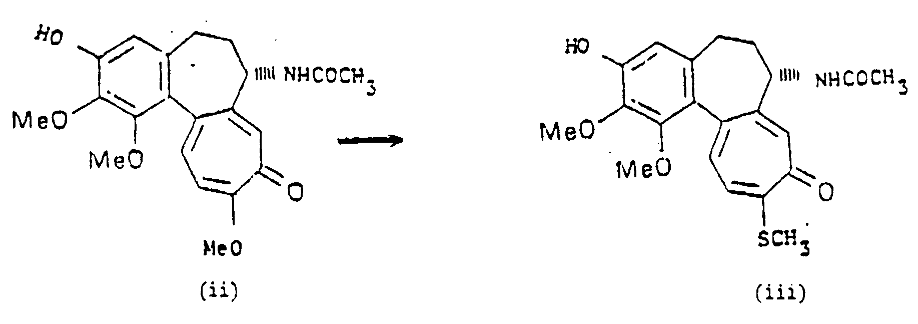
c) hydrolyzing 3-demethylthiocolchicine (iii) to remove the acetyl group in order to form compound (v), and 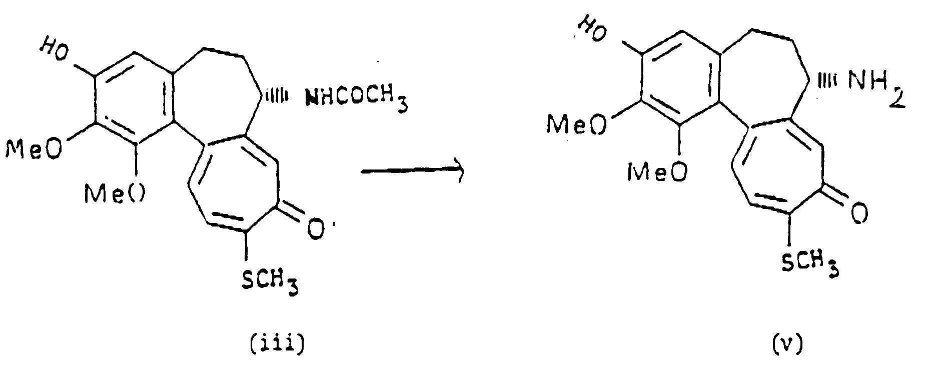
d) N-acylating and esterifying compound (v) to form an ester analog of 3-demethylthocolchicine (vi) 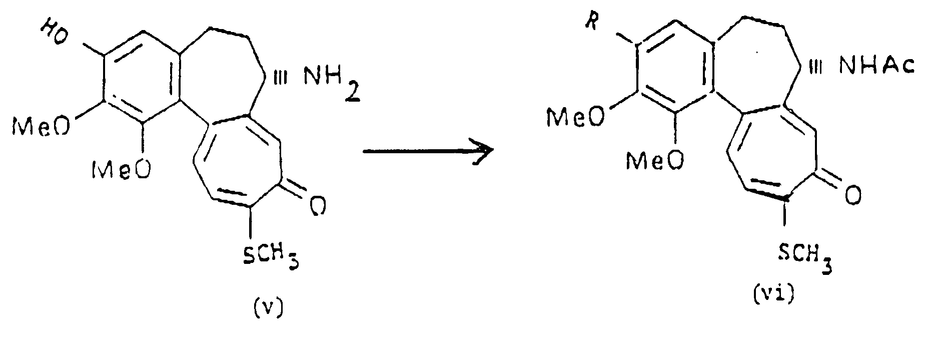 wherein R is an O-COOalkyl group the alkyl group having 1 to 4 carbon atoms, and the Ac group being acetyl or its higher homologs, haloacetyl, or alkoxyacetyl.

3. A method according to claim 1 or claim 2, wherein the product is 3-ethoxycarbonyl-3-demethylthiocolchicine.

4. A method according to claim 2, wherein the product is 3-butoxycarbonyl-3-demethylthiocolchicine.

5. A method according to any preceding claim which includes the further step of mixing a pharmaceutically effective amount of the product with a pharmaceutically acceptable carrier therefor to form a pharmaceutical composition.

## Patentansprüche (Patentansprüche für folgende(n) Vertragsstaat(en): AT, BE, CH, DE, FR, GB, IT, LI, LU, NL, SE)

1. Esterverbindung von 3-Demethylthiocolchicin oder ein N-Acylanaloges hiervon mit der Formel 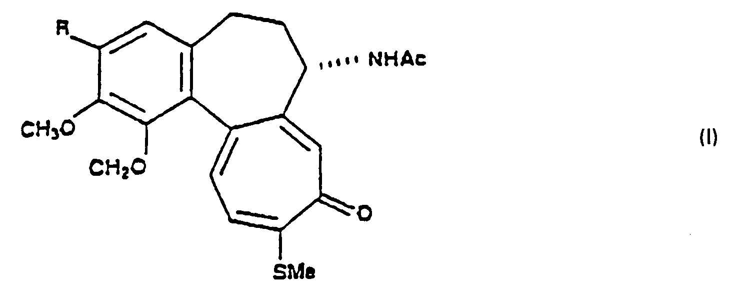 worin R eine O-COO-Alkylgruppe, deren Alkylgruppe 1 bis 4 Kohlenstoffatome hat, ist und die Ac-Gruppe Acetyl oder dessen höhere Homologe, Halogenacetyl oder Alkoxyacetyl ist.

2. Verbindung nach Anspruch 1, die 3-Ethoxycarbonyl-3-demethylthiocolchicin ist.

3. Verbindung nach Anspruch 1, die 3-Butoxycarbonyl-3-demethylthiocolchicin ist.

4. Verbindung nach einem der vorausgehenden Ansprüche für die Verwendung bei der Behandlung von Gicht.

5. Verbindung nach einem der Ansprüche 1 bis 3 für die Verwendung bei der Behandlung von Mittelmeerfieber.

6. Verbindung nach einem der Ansprüche 1 bis 3 für die Verwendung bei der Behandlung von Lebererkrankungen.

7. Verbindung nach Anspruch 6 für die Verwendung bei der Behandlung von Zirrhose, Tumorwachstum oder Entzündung der Leber.

8. Pharmazeutische Zusammensetzung, die eine pharmazeutisch wirksame Menge einer Verbindung nach einem der vorausgehenden Ansprüche und einen pharmazeutisch verträglichen Träger hierfür umfaßt.

9. Pharmazeutische Zusammensetzung nach Anspruch 8 in injizierbarer Form.

10. Pharmazeutische Zusammensetzung nach Anspruch 8 in oral verabreichbarer Form.

11. Pharmazeutische Zusammensetzung nach Anspruch 8 in der Form eines Cremes oder einer Salbe.

12. Zusammensetzung nach einem der Ansprüche 8 bis 11 in Dosierungseinheiten von 0,5 bis 10 mg/kg Körpergewicht des Patienten.

13. Verfahren zur Herstellung einer Esterverbindung von 3-Demethylthiocholchicin, bei dem man
a) Colchicosid (i) mit Phosphorsäure unter Bildung von 3-Demethylcolchicin (ii) umsetzt, 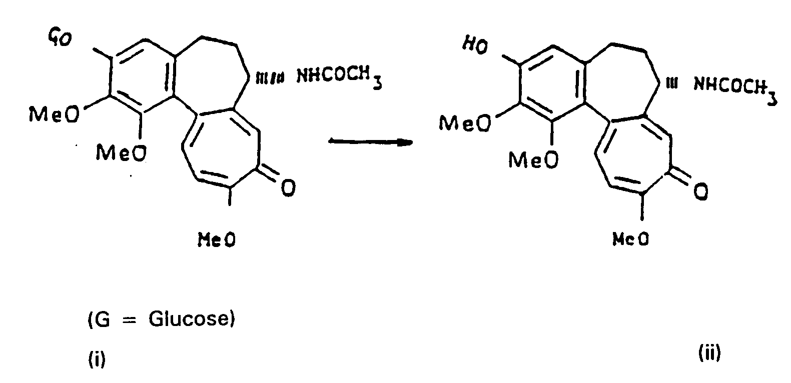
b) 3-Demethylcolchicin (ii) mit einem Alkalisalz von Methylmercaptan und Wasser bei Raumtemperatur unter Bildung von 3-Demethylthiocolchicin (iii) umsetzt 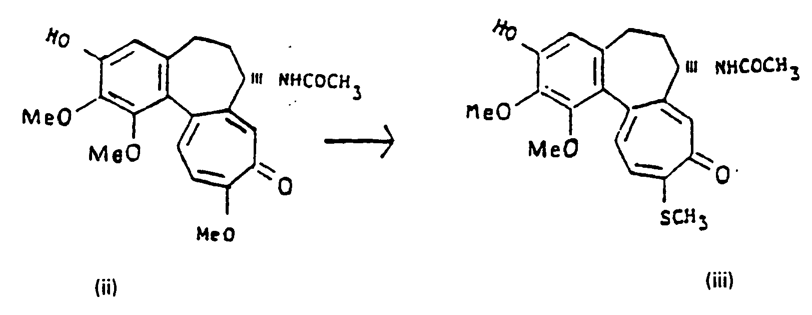 und
c) 3-Demethylthiocolchicin (iii) unter Bildung eines Esteranalogen hiervon (iv) verestert 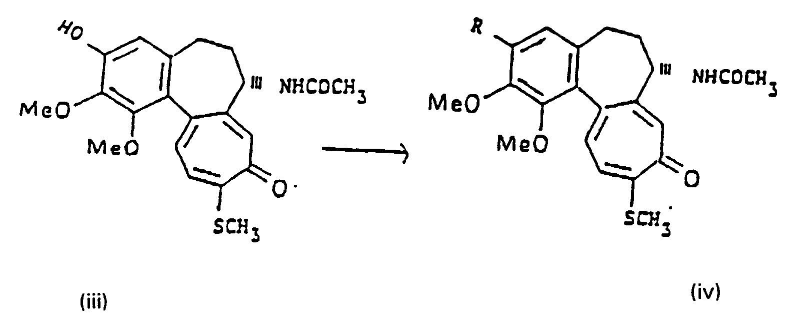 worin R eine O-COO-Alkylgruppe ist, wobei die Alkylgruppe 1 bis 4 Kohlenstoffatome hat.

14. Verfahren zur Herstellung einer Verbindung nach Anspruch 1, bei dem man
a) Colchicosid (i) mit Phosphorsäure unter Bildung von 3-Demethylcolchicin (ii) umsetzt 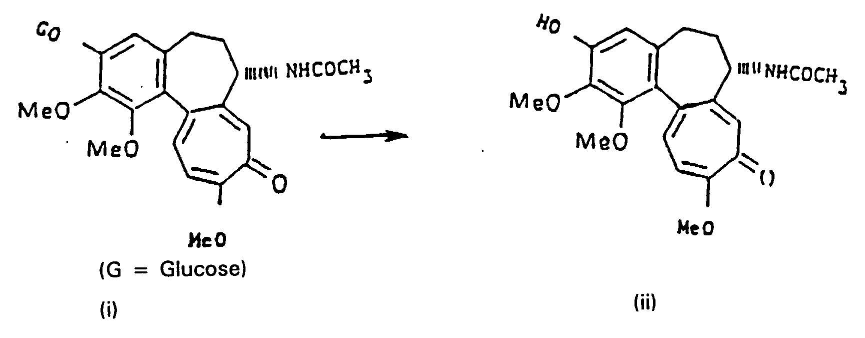
b) 3-Demethylcolchicin (ii) mit einem Alkalisalz von Methylmercaptan und Wasser bei Raumtemperatur unter Bildung von 3-Demethylthiocolchicin (iii) umsetzt 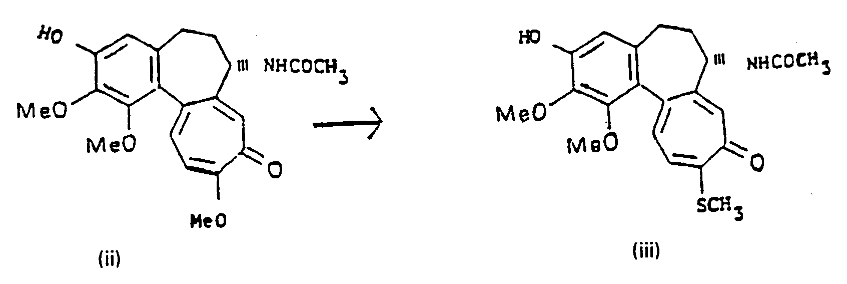
c) 3-Demethylthiocolchicin (iii) unter Entfernung der Acetylgruppe und Bildung der Verbindung (v) hydrolysiert 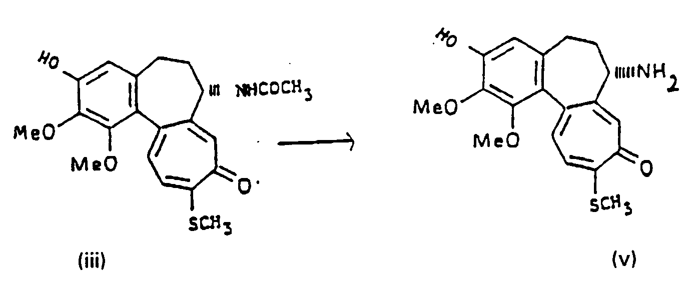 und
d) die Verbindung (v) unter Bildung eines Esteranalogen von 3-Demethylthiocolchicin (vi) N-acyliert und verestert 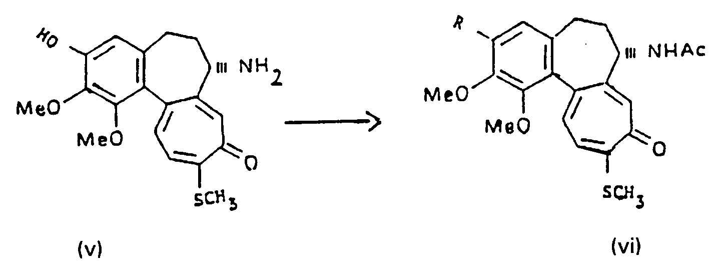 worin R eine O-COO-Alkylgruppe ist, wobei die Alkylgruppe 1 bis 4 Kohlenstoffatome hat, und die Gruppe Ac Acetyl oder höhere Homologe desselben, Halogenacetyl oder Alkoxyacetyl ist.

## Patentansprüche (Patentansprüche für folgende(n) Vertragsstaat(en): ES, GR)

1. Verfahren zur Herstellung eines Esters von 3-Demethylthiocolchicin, bei dem man
a) Colchicosid (i) mit Phosphorsäure unter Bildung von 3-Demethylcolchicin (ii) umsetzt 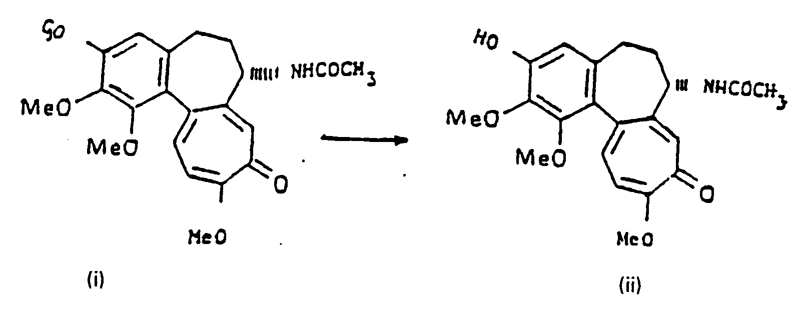
b) 3-Demethylcolchicin (ii) mit einem Alkalisalz von Methylmercaptan und Wasser bei Raumtemperatur unter Bildung von 3-Demethylthiocolchicin (iii) umsetzt 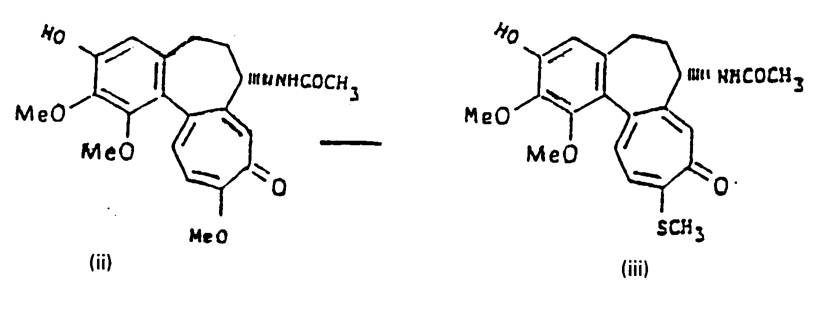 und
c) 3-Demethylthiocolchicin (iii) unter Bildung eines Esteranalogen hiervon (iv) verestert 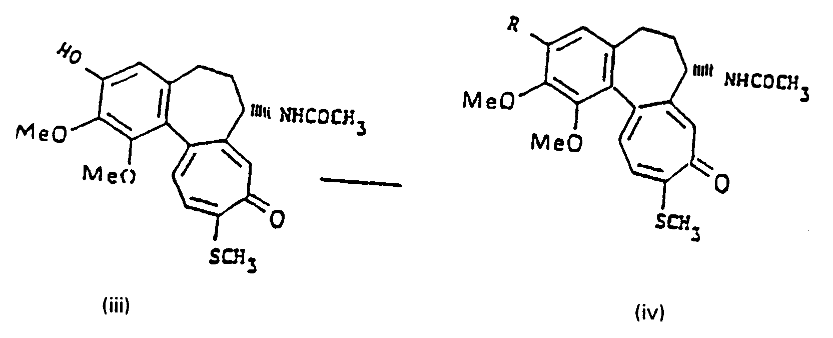 worin R eine O-COO-Alkylgruppe ist, worin die Alkylgruppe 1 bis 4 Kohlenstoffatome hat.

2. Verfahren zur Herstellung eines Esters von 3-Demethylthiocolchicin, bei dem man
a) Colchicosid (i) mit Phosphorsäure unter Bildung von 3-Demethylcolchicin (ii) umsetzt 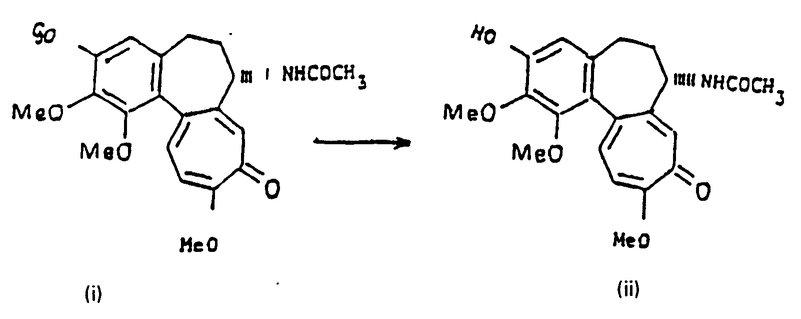
b) 3-Demethylcolchicin (ii) mit einem Alkalisalz von Methylmercaptan und Wasser bei Raumtemperatur unter Bildung von 3-Demethylthiocolchicin (iii) umsetzt 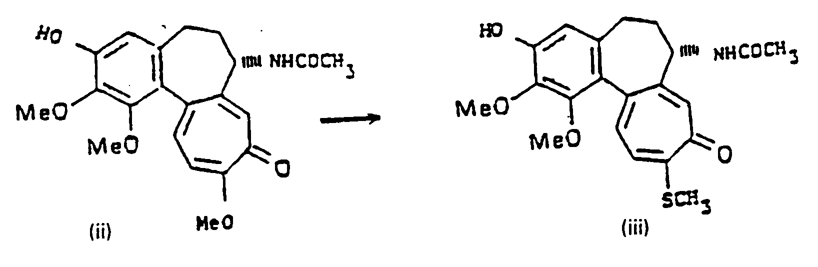
c) 3-Demethylthiocolchicin (iii) unter Entfernung der Acetylgruppe und Bildung der Verbindung (v) hydrolysiert 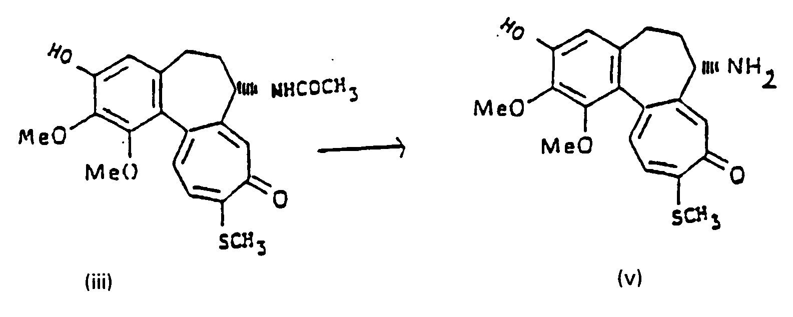 und
d) die Verbindung (v) unter Bildung eines Esteranalogen von 3-Demethylthiocolchicin (vi) N-acyliert und verestert 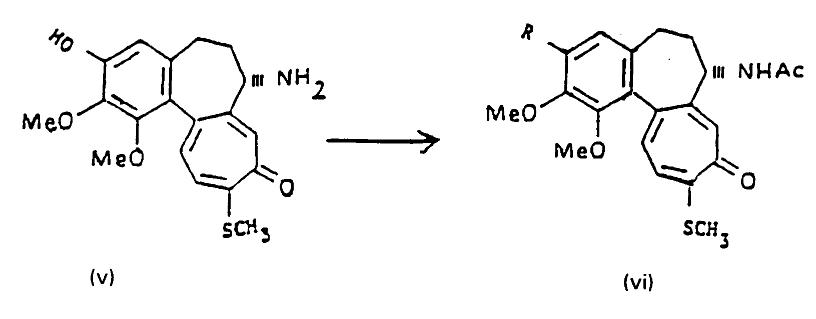 worin R eine O-COO-Alkylgruppe ist, worin die Alkylgruppe 1 bis 4 Kohlenstoffatome hat, und die Ac-Gruppe Acetyl oder deren höhere Homologe, Halogenacetyl oder Alkoxyacetyl ist.

3. Verfahren nach Anspruch 1 oder Anspruch 2, bei dem das Produkt 3-Ethoxycarbonyl-3-demethylthiocolchicin ist.

4. Verfahren nach Anspruch 2, bei dem das Produkt 3-Butoxycarbonyl-3-demethylthiocolchicin ist.

5. Verfahren nach einem der vorausgehenden Ansprüche, welches die weitere Stufe eines Vermischens einer pharmazeutisch wirksamen Menge des Produktes mit einem pharmazeutisch verträglichen Träger hierfür unter Bildung einer pharmazeutischen Zusammensetzung einschließt.

## Revendications (Revendications pour l'(les) Etat(s) contractant(s) suivant(s): AT, BE, CH, DE, FR, GB, IT, LI, LU, NL, SE)

1. Ester de la 3-déméthylthiocolchicine ou d'un analogue N-acylé de celle-ci répondant à la formule : 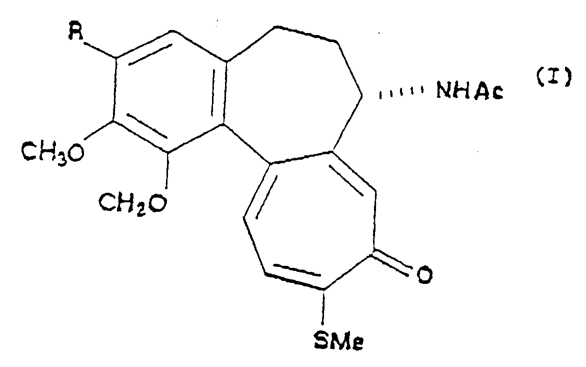 dans laquelle R est un groupe O-COOalkyle, le groupe alkyle ayant 1 à 4 atomes de carbone et le groupe Ac est un acétyle ou ses homologues supérieurs, un haloacétyle ou un alkoxyacétyle.

2. Composé selon la revendication 1, caractérisé en ce qu'il s'agit de la 3-éthoxycarbonyl-3-déméthylthiocolchicine.

3. Composé selon la revendication 1, caractérisé en ce qu'il s'agit de la 3-butoxycarbonyl-3-déméthylthiocolchicine.

4. Composé selon l'une quelconque des revendications précédentes pour le traitement de la goutte.

5. Composé selon l'une quelconque des revendications 1 à 3 pour le traitement de la fièvre méditerranéenne.

6. Composé selon l'une quelconque des revendications 1 à 3, pour le traitement de désordres du foie.

7. Composé selon la revendication 6, pour le traitement de la cirrhose, de la croissance de tumeur ou de l'inflammation du foie.

8. Composition pharmaceutique, caractérisée en ce qu'elle comprend une quantité efficace d'un composé selon l'une quelconque des revendications précédentes et un véhicule pharmaceutiquement acceptable pour celui-ci.

9. Composition pharmaceutique selon la revendication 8 sous forme injectable.

10. Composition pharmaceutique selon la revendication 8, sous forme administrable par voie orale.

11. Composition pharmaceutique selon la revendication 8, sous forme d'une crème ou d'une pommade.

12. Composition selon l'une quelconque des revendications 8 à 11, sous forme de dosages unitaires de 0,5 à 10 mg/kg de poids corporel du patient.

13. Procédé de préparation d'un ester de la 3-déméthylthiocolchicine, caractérisé en ce qu'il comprend:
a) la réaction du colchicoside (i) avec de l'acide phosphorique pour former la 3-déméthylcolchicine (ii) 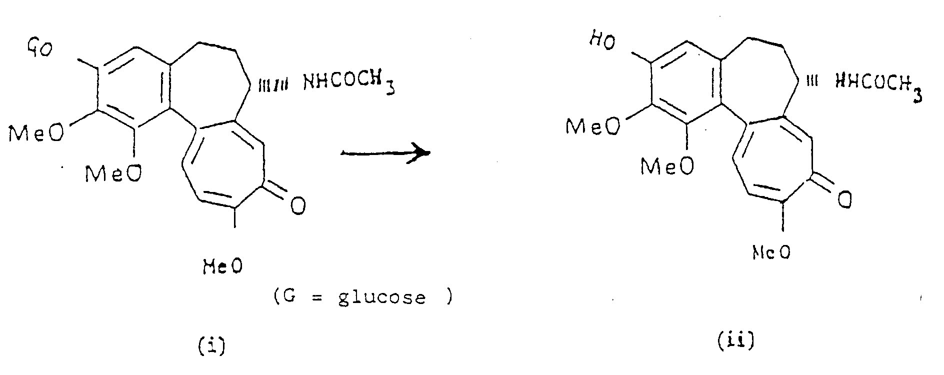
b) la réaction de la 3-déméthylcolchicine (ii) avec un sel alcalin de méthyl mercaptan et de l'eau à température ambiante pour former la 3-déméthlylthiocolchicine (iii), et 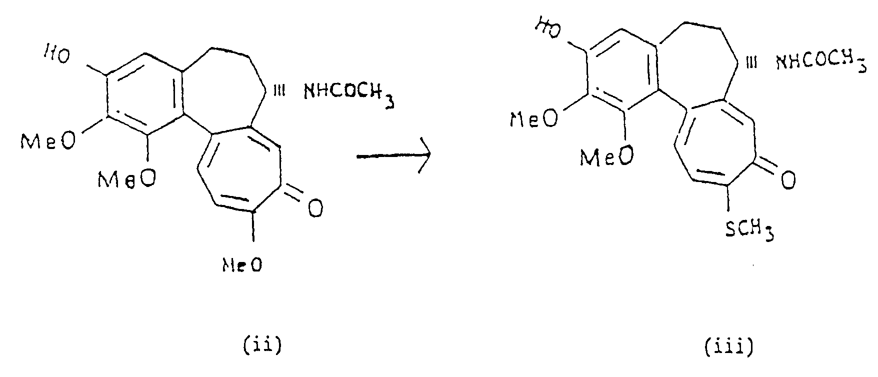
c) l'estérification de la 3-déméthylthiocolchicine (iii) pour former un analogue ester de celle-ci (iv). 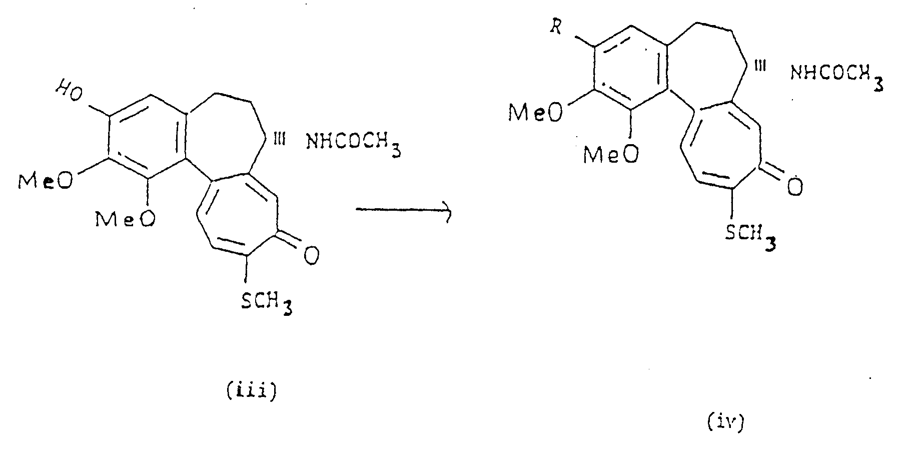 où R représente un groupe O-COOalkyle, le groupe alkyle ayant de 1 à 4 atomes de carbone.

14. Procédé de préparation d'une composition selon la revendication 1, caractérisé en ce qu'il comprend :
a) la réaction du colchicoside (i) avec de l'acide phosphorique pour former la 3-déméthylcolchicine (ii) 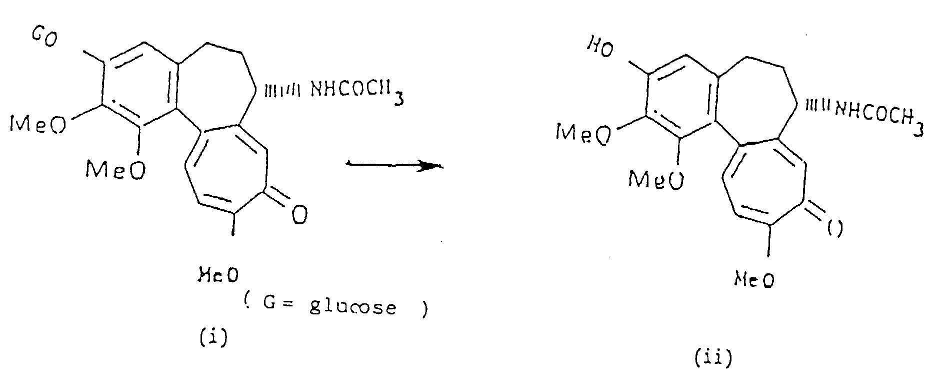
b) la réaction de la 3-déméthylcolchicine (ii) avec un sel alcalin de méthyl mercaptone et de l'eau à température ambiante pour former la 3-déméthylthiocolchicine (iii) 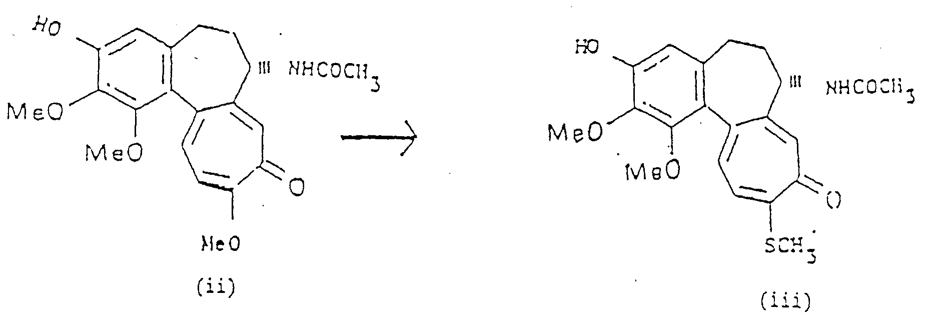
c) l'hydrolyse de la 3-déméthylthiocolchicine (iii) pour éliminer le groupe acétyle afin de former le composé (v), et, 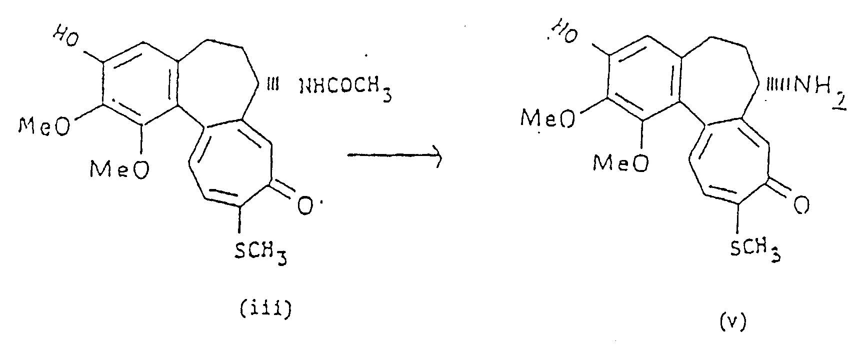
d) la N-acylation et estérification du composé (v) pour former un analogue ester de la 3-déméthylthiocolchicine (vi) 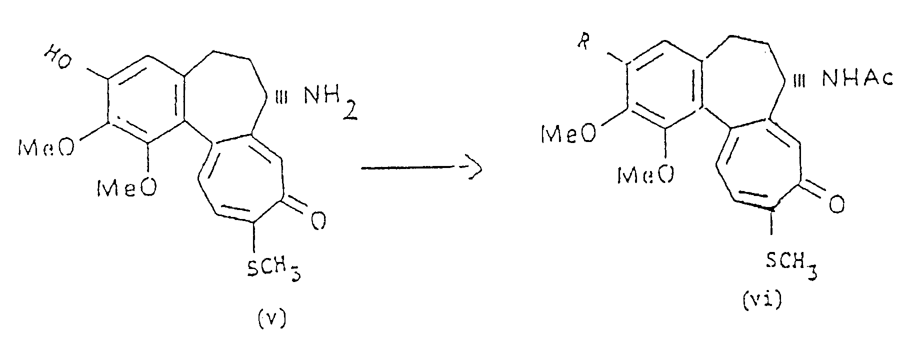 où R est un groupe O-COOalkyle, le groupe alkyle ayant 1 à 4 atomes de carbone, et le groupe Ac est un acétyle ou ses homologues supérieurs, un haloacétyle ou un alkoxyacétyle.

## Revendications (Revendications pour l'(les) Etat(s) contractant(s) suivant(s): ES, GR)

1. Procédé de préparation d'un ester de 3-déméthylthiocolchicine, caractérisé en ce qu'il comprend:
a) la réaction du colchicoside (i) avec de l'acide phosphorique pour former la 3-déméthylcolchicine (ii) 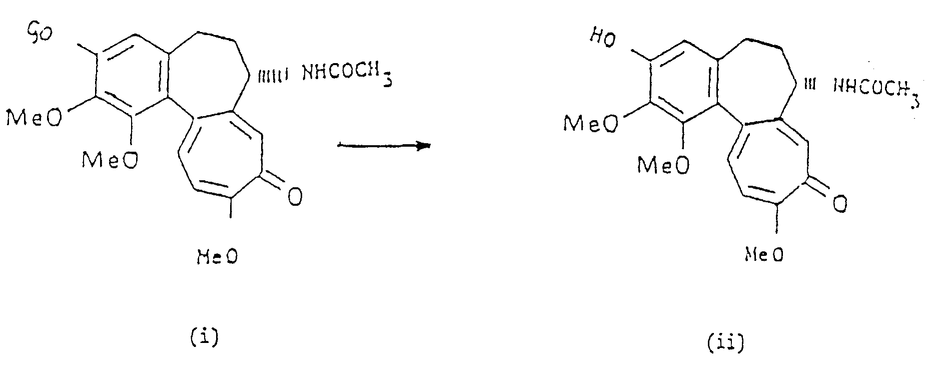
b) la réaction de la 3-déméthylcolchicine (ii) avec un sel alcalin de méthyl mercaptan et de l'eau à température ambiante pour former la 3-déméthylthiocolchicine (iii), et 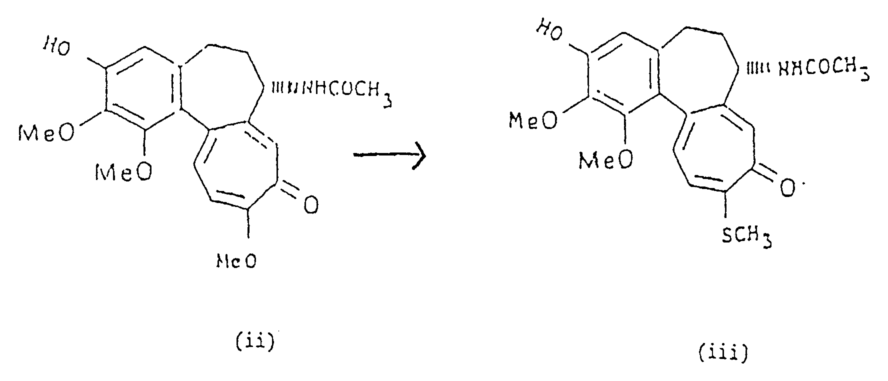
c) l'estérification de la 3-déméthylthiocolchicine (iii) pour former un analogue ester de celle-ci (iv) 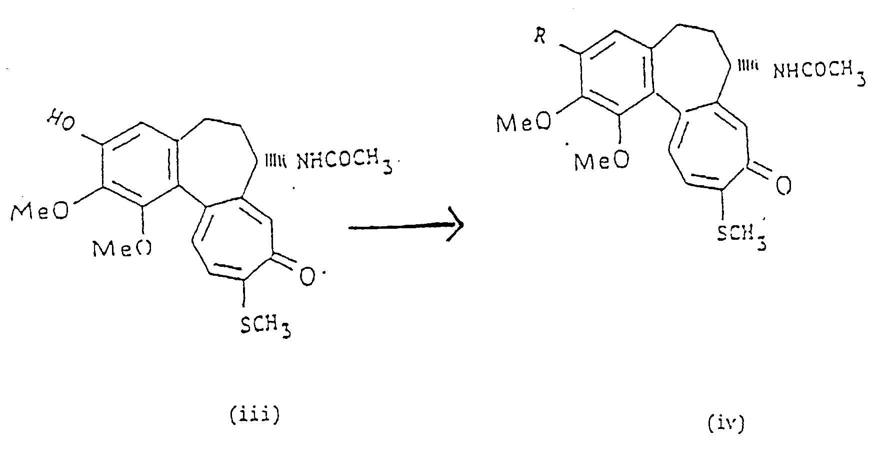 où R représente un groupe O-COOalkyle, le groupe alkyle ayant de 1 à 4 atomes de carbone.

2. Procédé de préparation d'un ester de 3-déméthylthiocolchicine, caractérisé en ce qu'il comprend:
a) la réaction du colchicoside (i) avec de l'acide phosphorique pour former la 3-déméthylcolchicine (ii) 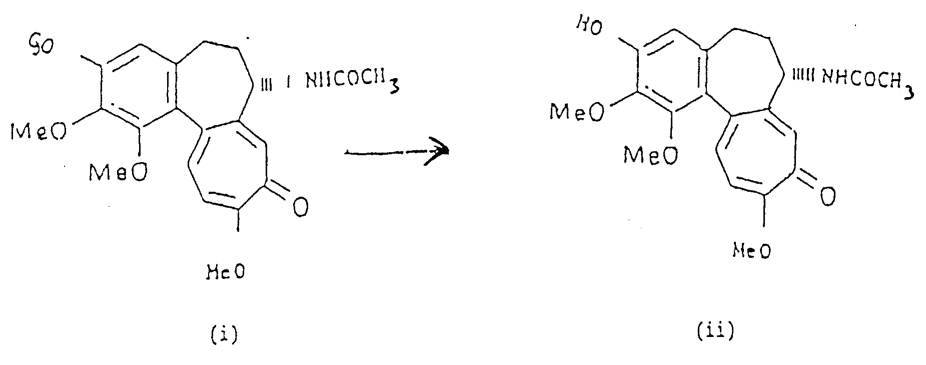
b) la réaction de la 3-déméthylcolchicine (ii) avec un sel alcalin de méthyl mercaptone et de l'eau à température ambiante pour former la 3-déméthylthiocolchicine (iii) 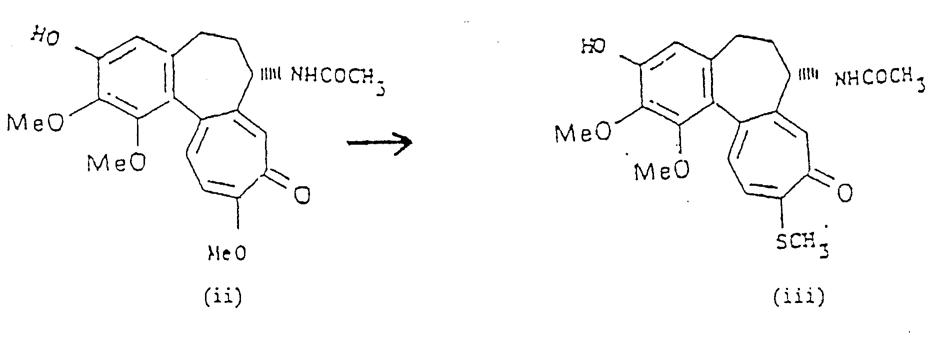
c) l'hydrolyse de la 3-déméthylthiocolchicine (iii) pour éliminer le groupe acétyle afin de former le composé (v), et, 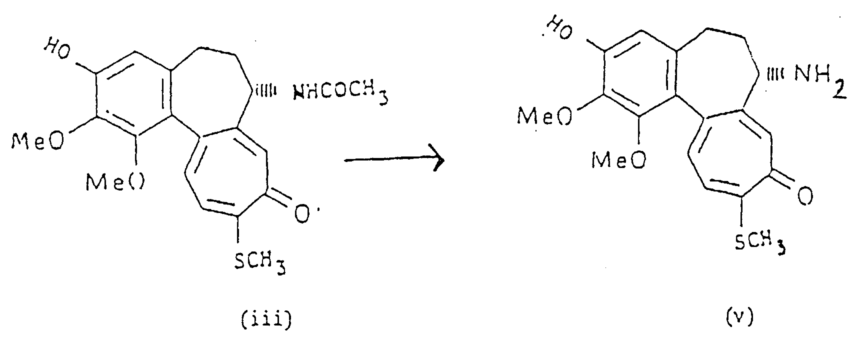
d) la N-acylation et esterification du composé (v) pour former un analogue ester de la 3-déméthylthiocolchicine (vi) 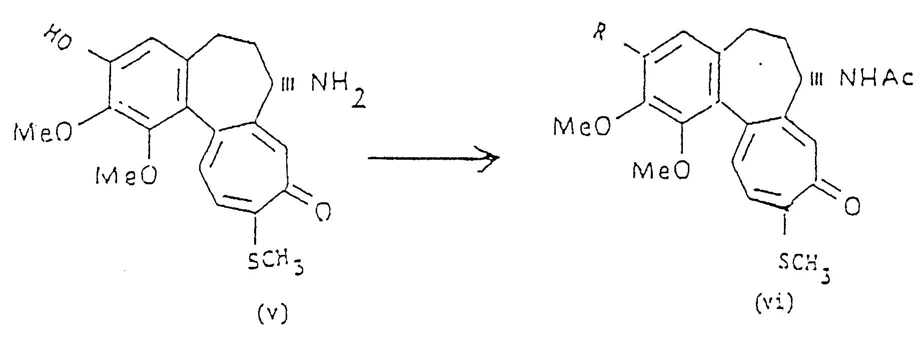 où R est un groupe O-COOalkyle, le groupe alkyle ayant 1 à 4 atomes de carbone, et le groupe Ac est un acétyle ou ses homologues supérieurs, un haloacétyle ou un alkoxyacétyle.

3. Procédé selon la revendication 1 ou 2, caractérisé en ce que le produit est la 3-éthoxycarbonyle-3-déméthylthiocolchicine.

4. Procédé selon la revendication 2, caractérisé en ce que le produit est la 3-butoxycarbonyl-3-déméthylthiocolchicine.

5. Procédé selon l'une quelconque des revendications précédentes caractérisé en ce qu'il comprend en outre une étape de mélange d'une quantité pharmaceutiquement efficace du produit avec un véhicule pharmaceutiquement acceptable pour celui-ci pour former une composition pharmaceutique.
